# EUROPEAN PATENT SPECIFICATION

(11) **EP 2 497 407 B1**
(45) Date of publication and mention of the grant of the patent: **14.12.2016**
(21) Application number: 10826690.9
(22) Date of filing: 26.10.2010
(51) Int. Cl.: A61B 1/267, A61B 1/06

(54) **PHARYNX EXPANDER**
RACHENWEITUNGSVORRICHTUNG
EXPANSEUR DE PHARYNX

(30) Priority: 02.11.2009 JP 2009252484
(43) Date of publication of application: 12.09.2012
(73) Proprietor: Nagoya City University, Aichi 467-8601 (JP)
(72) Inventor: KUNIMOTO Katsushi, Nagoya-shi Aichi 464-0083 (JP)
(74) Representative: Garavelli, Paolo
(86) International application number: PCT/JP2010/068905
(87) International publication number: WO 2011/052558

(56) References cited:
- EP-A1- 1 025 796
- GB-A- 375 491
- GB-A- 375 491
- JP-A- 10 509 614
- JP-A- 2006 517 120
- US-A- 4 384 570
- US-A- 4 384 570
- US-A- 4 947 896
- US-A- 5 542 905
- US-A- 5 571 071
- US-A1- 2005 090 712
- US-A1- 2007 093 693
- US-B1- 6 494 828
- US-B1- 6 569 089

## Description

### [Technical Field]

The present invention relates to improvement of a larynx expander.

### [Background Art]

A larynx expander is inserted into a larynx of a patient to expand the larynx in emergency and the like, thereby securing an airway or inserting an intubation tube into the larynx.

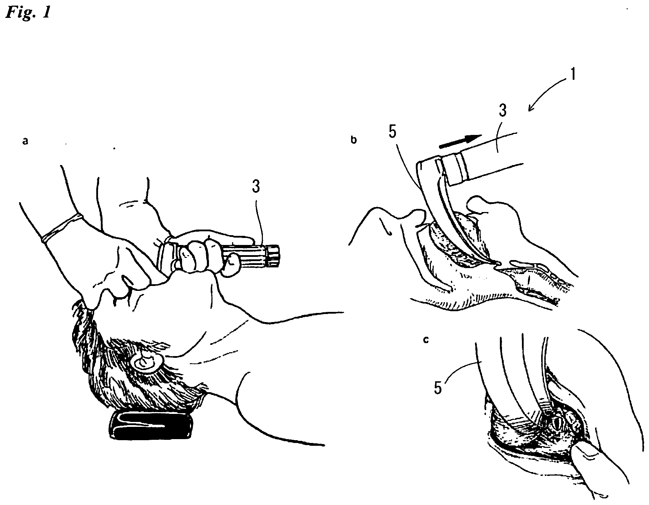

Conventionally, a laryngoscope 1 shown in Fig. 1 has been used as one of larynx expanders. The laryngoscope 1 has a structure, in which a grip 3 in a circular column shape and a blade 5 are connected in an L-shape. The blade 5 is inserted into a larynx, and the grip 3 is moved in its axial direction to secure an airway of a patient. Further, an intubation tube is inserted (refer to Non-patent document 1).

There has been proposed a laryngoscope having a light source attached to its blade to improve operability (refer to Patent document 1).

### [Prior Technical Documents]

### [Patent document]

US-A1-2007/093693, GB-A-375491 and US-A-4 384 570 siclose larynx expanders according to the preamble of Claim 1.

JP-A-2009-500056
JP-A-2009-500056

### [Non-patent document]

[Non-patent document 1] Monthly SHOBOH, April 2003 issue Further, document EP 1 025 796 discloses a larynx expander according to the preamble of claim 1.

### [Disclosure of the Invention]

### [Problems to be solved by the Invention]

The laryngoscope has a long history of use. However, the basic shape constituted by the grip 3 in the circular column shape and the blade 5 attached to the end of the grip 3 has not been changed at all.

Generally, the laryngoscope is used for an unconscious patient. Therefore, (1) the blade 5 is inserted to the larynx through a firmly closed mouth of the patient, and (2) a lower jaw is opened to secure the airway.

Specifically in the (2) procedure to open the lower jaw, an operator grips the grip 3 firmly and moves the grip 3 in its axial direction. At that time, since a palm grasps the grip 3 in a lateral direction, fingers cannot catch the grip 3 when moving the grip 3 in its axial direction, so the fingers can slip easily. Accordingly, large grip strength is required to hold the grip 3, thereby putting a large load on the operator.

### [Means for solving the Problems]

The present invention has been made to solve the above-described problem. A first aspect of the disclosure is defined as follows.

That is, a larynx expander has a grip and a blade. The grip has a first grip section on a blade side and a second grip section on a free end side. The first grip section is thinner than the second grip section. A stepped section between the first grip section and the second grip section is formed on a backside of the grip. A free end of the second grip section has a swollen section on its belly side.

In the thus-constructed larynx expanding section, the first grip section on the blade side is formed to be thinner than the second grip section in the grip. Therefore, the stepped section is formed in the connection between the first grip section and the second grip section. The stepped section is provided on the backside of the grip (i.e., on side opposite to protruding direction of blade). Thus, when the grip is gripped in the lateral direction, the finger(s) or the palm engages with the stepped section, whereby the force can be applied to the grip easily.

Further, the swollen section is provided on the belly side of the free end of the second grip section. Therefore, the finger(s) can catch also the swollen section to prevent the slip of the grip.

Since the stepped section is formed on the backside of the grip and the swollen section is formed on the belly side of the free end of the grip, the finger(s) can catch the swollen section while pressing substantially a central portion of the palm against the stepped section. Thus, when the grip is moved in its axial direction, the central portion of the palm and the side surface of the finger(s) of the operator engage with the grip. Accordingly, the force can be applied efficiently.

A second aspect of the disclosure is defined as follows. That is, in the larynx expander according to the first aspect, the grip is formed in an arc-like shape having a belly side that constitutes an inner periphery of the grip.

In the thus-defined larynx expander according to the second aspect, the grip is formed in the arc-like shape having the belly side that constitutes the inner periphery of the grip. Therefore, the grip faces downward when the blade is inserted. During the procedure, it is necessary to apply a force in the axial direction of the blade. Therefore, if the grip faces downward in this way, the operator can use its own weight efficiently to lower the grip and to expand the larynx of the patient, thereby alleviating the load during the procedure.

A third aspect of the disclosure is defined as follows. That is, in the larynx expander defined in the first or second aspect, a first light source is aligned in the swollen section of the second grip section.

When the operator inserts the blade into the mouth cavity first during the use of the larynx expander, the operator grasps a portion of the grip close to the blade, i.e., the first grip section, with one hand and touches the patient with the other hand. Therefore, with the larynx expander according to the above-described third aspect, the second grip section is free, and the first light source of the swollen section of the second grip section is exposed. The swollen section is formed on the belly side of the second grip section, i.e., in the protruding direction of the blade. Therefore, in the above-described early stage of the procedure, the light emitted from the first light source of the swollen section is emitted to the blade side, i.e., to the face of the patient. Therefore, the mouth of the patient can be illuminated brightly in the early stage of the procedure. Thus, the procedure can be performed smoothly when the procedure using the larynx expander is performed on a site of an accident or a disaster occurring during the night.

A fourth aspect of the disclosure is defined as follows. That is, in the larynx expanding section according to the third aspect, a second light source is aligned near a portion on the belly side of the first grip section connected to the blade.

Since the blade is inserted into the larynx in a middle stage of the procedure, it is preferable to illuminate the inside of the mouth cavity and the larynx. Therefore, by arranging the second light source in a root portion of the first grip section on the belly side (blade protruding surface side), i.e., in a portion close to the portion of the first grip section connected to the blade, as in the larynx expanding section defined in the fourth aspect, the inside of the mouth cavity and the larynx can be illuminated with the light from the second light source. Since the operator holds the second grip section at that time, the second light source of the first grip section is not obstructed.

The blade itself may emit the light to illuminate the inside of the mouth cavity and the larynx of the patient (fifth aspect).

That is, a fifth aspect of the disclosure is defined as follows.

In the larynx expander defined in the fourth aspect, the blade is made of a light guiding body. The grip has a third light source that faces the blade to emit the light into the blade. The light emitted into the blade is discharged from a peripheral surface of the blade.

The entirety of the blade may emit the light or only a part of the blade may emit the light.

A sixth aspect of the disclosure is defined as follows.

In the larynx expander according to the fifth aspect, the blade is joined to the grip such that the blade is detachable or foldable. At least one of the first to third light sources is switched on when the blade is set upright to the grip in a used state. The first to third light sources are switched off when the blade is detached from the grip or is folded.

With the thus-defined larynx expander according to the sixth aspect, all of the first to third light sources are switched off when the blade is separated from the grip or is folded in the unused state. At least one of the first to third light sources is switched on when the blade is brought to the upright state to the grip in a used state.

Thus, the light sources are surely switched off when the larynx expander is not used. Accordingly, electric power consumption can be suppressed. Specifically, it is necessary to equip the larynx expander used in the disaster-stricken area or the like with the light source and a power supply of the light source is insufficient. Therefore, the on-off control of the light sources as in the larynx expanding section according to the sixth aspect is preferable also from a viewpoint of maintenance.

A seventh aspect of the disclosure is defined as follows. That is, in the larynx expander according to any one of third to sixth aspects, the second grip section incorporates a power supply.

In the thus-defined larynx expander according to the seventh aspect, the second grip section is formed to be thicker than the first grip section. Therefore, the power supply arranged inside the second grip section can secure a large capacity. Therefore, the light source section can secure a sufficient operating time, thereby providing the larynx expander suitably used in the disaster-stricken area and the like.
The invention is defined in the claims. Other embodiments are merely exemplary.

### [Brief Description of the Drawings]

[Fig. 1] Fig. 1 is an illustration showing a procedure using a conventional laryngoscope.
[Fig. 2] Fig. 2 is a side view showing a larynx expander in a used state according to an embodiment of the present invention.
[Fig. 3] Fig. 3 is a side view showing the larynx expander in an unused state.

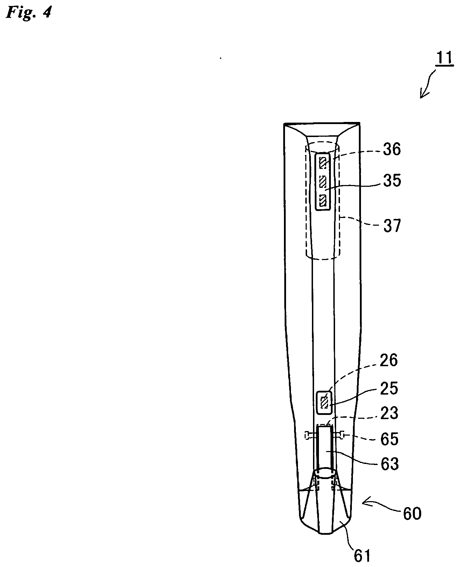

[Fig. 4] Fig. 4 is a front view showing the larynx expander in the used state.
[Fig. 5] Fig. 5 is a perspective view showing the larynx expander, and part (A) shows the used state and part (B) shows the unused state.
[Fig. 6] Fig. 6 is a block diagram showing an electric system of the larynx expander.
[Fig. 7] Fig. 7 is a schematic diagram illustrating a state where a face of a patient is illuminated with a first LED light source in an early stage of the procedure using the larynx expander.
[Fig. 8] Fig. 8 is a schematic diagram illustrating a state where inside of a mouth cavity is illuminated with a second LED light source and the like in a middle stage of the procedure using the larynx expander.
[Fig. 9] Fig. 9 is a schematic diagram showing a state where the larynx is expanded in a final stage of the procedure.

Hereinafter, the present invention will be explained in more details based on an embodiment.

A larynx expander 11 according to the embodiment has a grip 20 and a blade 60.

The grip 20 has a first grip section21 on a blade 60 side and a second grip section 31 on a free end side.

The first grip section 21 is thinner than the second grip section 31. Accordingly, a step is formed in a connection between the first and second grip sections 21, 31. In this example, the step 40 is formed on a backside of the grip 20. The first grip section 21 becomes thick gradually from a connection between the first grip section 21 and the blade 60. The first grip section 21 provides the step 40 in the connection between the first and second grip sections 21, 31.

A groove 23 is formed on an end portion of the first grip section 21 joined with the blade 60, the groove 23 extending from an end surface of the end portion. A window 25 is formed near the groove 23 on a belly side. A second LED light source (second light source) 26 is accommodated inside the window 25.

The second grip section 31 is formed to be thicker than the first grip section 20. A swollen section 33 is formed on a belly side of the free end of the second grip section 31. A window 35 is formed in the swollen section 33, and a first LED light source 36 (first light source) is accommodated inside the window 35. A power supply case 37 is provided in the second grip section 31. A battery as a power supply is set inside the power supply case 37.

A base section and the swollen section of the second grip section may be separate bodies.

The first grip section 21 and the second grip section 31 are formed as an integral body in an arc-like shape having a belly side (i.e., protruding side of blade 60) that constitutes an inner periphery of the grip.

A material for forming the grip 11 may be metal or hard plastic.

The blade 60 has a blade main body 61, which is in a claw-like shape as in the prior art, and a connection 63. In this example, the entire body of the blade 60 is made as an integral body from a translucent material such as an acrylic resin. Thus, the light incoming through the connection 63 repeats diffused reflection inside and is discharged from the entire peripheral surface of the blade main body 61.

In the above construction, the connection 63 is a plate-like member that is set upright at a center of a base end section of the blade main body 61. The connection 63 is inserted into the groove 23 formed in the lower end of the grip 11. A reference numeral 65 represents a hinge shaft for attaching the connection 63 to the lower end of the grip 11 such that the connection 63 can swing.

A third LED light source 66 (third light source) is arranged on a peripheral wall of the groove 23 and faces a side surface of the connection 63 to emit the light to the side surface. A light source facing an end surface of the connection 63 may be provided.

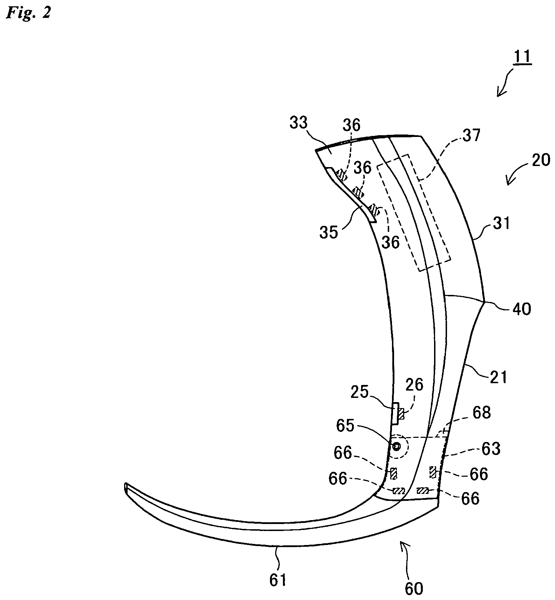

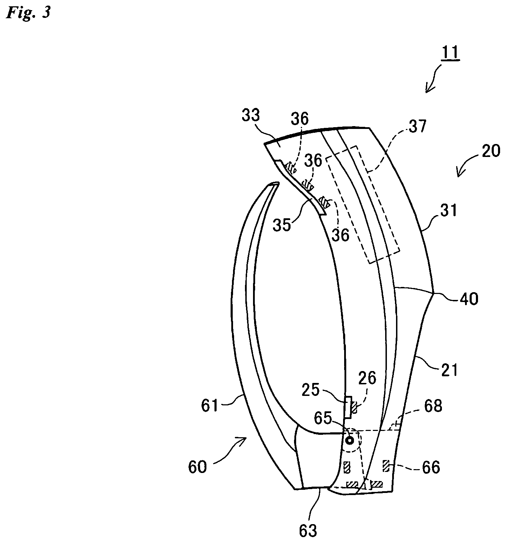

A push-type switch 68 is arranged on an upper wall of the groove 23. As shown in Fig. 2, in a used state where the blade 60 is opened and is set upright to the grip 11, an upper edge of the connection 63 interferes with the switch 68 and switches on the switch 68. In an accommodated state in which the blade 60 is folded as shown in Fig. 3, the upper edge of the connection 63 separates from the switch 68 and switches off the switch 68. When the switch 68 is switched on, the first to third LED light sources are lit. When the switch 68 is switched off, the first to third LED light sources are unlit.

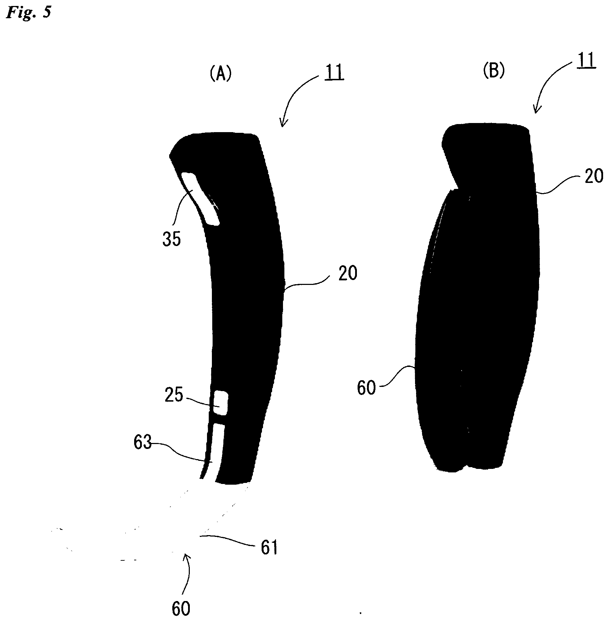

Fig. 5(A) shows a lit state of the larynx expander 11 and Fig. 5(B) shows an unlit state of the larynx expander 11.

As shown in Fig. 5(A), the groove 23 is formed in the lower end of the grip 20, and a connection 63 made of a light guiding body is fitted to the groove 23. Thus, the light is emitted from a portion of the connection 63 (connection light emitting section), which is exposed out of the groove 23. The connection light emitting section illuminates the inside of the mouth cavity and the larynx of the patient together with the second LED light source 25.

From such the viewpoint, when the groove is formed in the blade connection of the grip 20, it is preferable that the groove opens at least to the belly side of the grip 20 and a portion of the connection fitted to the groove is exposed out of the groove such that the exposed portion serves as a light emitting surface.

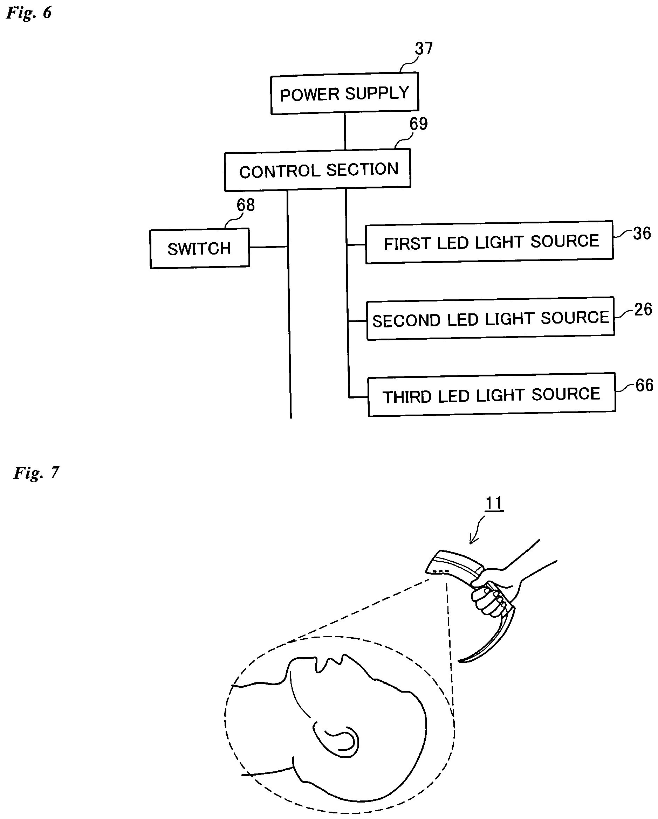

An electric system of the larynx expander 11 is shown in Fig. 6. Reference numeral 69 represents a control section. The control section 69 applies a power from the power supply 37 to intended one of the LED light sources 36, 26, 66 in response to an ON signal or an OFF signal from the limit switch 68.

In this example, the blade 60 is rotatably attached to the end of the grip 11. However, the blade may be detachable from the grip. In this case, when a used state is formed by attaching the blade to the grip, a part of the blade interferes with the switch and switches on the switch. When the blade is detached from the grip, the interference is dissolved and the switch is switched off.

In the above, a part or entirety of the first to third LED light sources may be omitted. Instead of lighting all the LED light sources in response to the switching on of the switch, only a part of the LED light sources may be lit.

Next, a mode of use of the larynx expander 11 will be explained based on Figs. 7 to 9.

If the larynx expander 11 is brought to the used state, the first to third LED light sources are lit. In the early stage of the procedure (refer to Fig. 7), it is necessary to check the state of the mouth of the patient. When the blade 60 is inserted into the mouth of the patient, the operator grasps the root side of the grip 11, i.e., the first grip section 21, with one hand (at this time, second light source is blocked), and holds the patient with the other hand. In this state, the swollen section 33 of the free end of the second grip section 31 is free. The mouth of the patient can be illuminated with the light from the first LED light source 36.

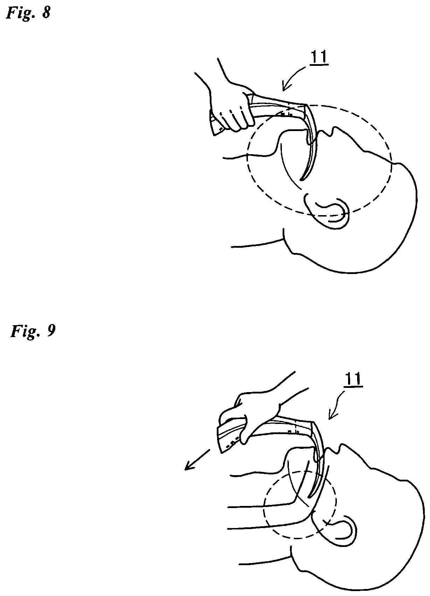

After the blade 60 is inserted into the mouth cavity of the patient, it is necessary to observe the inside of the mouth cavity and the larynx as shown in Fig. 8. At that time, the hand of the operator moves from the first grip section 21 to the second grip section 31. Therefore, the second LED light source 26 is uncovered. Accordingly, the light from the second LED light source 26, the light from the connection light source section and the light from the blade 60 brightly illuminate the inside of the mouth cavity and the larynx.

After the blade 60 is inserted into the larynx, the grip 11 is moved in a direction of an arrow mark in Fig. 9 to widen a jaw and the larynx, thereby securing an airway and enabling insertion of an intubation tube. At that time, the hand of the operator catches the step 41 and the swollen section 33 of the grip 11. Therefore, slip of the grip 11 can be inhibited and the force can be applied efficiently.

In this example, the entire blade 60 emits the light. Therefore, a state in a deep portion of the larynx can be also observed visually.

### [Brief Explanation of Reference Numerals]

1,11 LARYNX EXPANDER
3, 21 GRIP
5, 60 BLADE
21 FIRST GRIP SECTION
26 SECOND LED LIGHT SOURCE
31 SECOND GRIP SECTION
33 SWOLLEN SECTION
36 FIRST LED LIGHT SOURCE
37 POWER SUPPLY
41 STEP

## Claims

1. A larynx expander (11) comprising:
an arc-like shaped grip (20); and
a blade (60), wherein
the grip (20) has a distal grip section (21) connected with the blade (60) and a proximal grip section (31) on a proximal, free end side of the grip;
the distal grip section (21) being thinner than the proximal grip section (31), the distal grip section (21) becoming thick gradually from a connection between the distal grip section (21) and the blade (60),
the grip having a belly side, constituting the inner periphery of the grip and facing the blade when the blade is folded; and a backside, opposing the belly side; the free, proximal end of the proximal grip section (31) having a swollen section (33) on its belly side,
**characterized by** further comprising:
an angle stepped section (40) between the distal grip section (21) and the proximal grip section (31);
said angle stepped section (40) being formed solely on the backside of the grip; the angle stepped section (40) being adapted to be engaged by fingers or a palm of a hand of an operator to apply a force to the grip (20).

2. The larynx expander (11) as in claim 1, wherein
a first light source (36) is aligned in the swollen section of the proximal grip section (31).

3. The larynx expander (11) as in claim 2, wherein
a second light source (26) is aligned near a portion on the belly side of the distal grip section (21) connected to the blade (60).

4. The larynx expander (11) as in claim 3, wherein
the blade (60) is made of a light guiding body,
the grip (20) has a third light source (66) that faces the blade (60) to emit the light into the blade (60), so that
the light emitted into the blade (60) is discharged from a peripheral surface of the blade (60).

5. The larynx expander (11) as in claim 4, wherein
the blade (60) is joined to the grip (20) such that the blade (60) is detachable or foldable,
at least one of the first to third light sources (36, 26, 66) is switched on when the blade (60) is set upright to the grip (20) in a used state, and
the first to third light sources (36, 26, 66) are switched off when the blade (60) is detached from the grip (20) or is folded.

6. The larynx expander (11) as in any one of claims 3 to 5, wherein
the proximal grip section (31) incorporates a power supply (37).

## Patentansprüche

1. Kehlkopfdilatator (11) mit:
einem Griff (20), der eine bogenähnliche Form hat; und
einem Messer (60), dessen
Griff (20) einen distalen Griffabschnitt (21), der mit dem Messer (60) verbunden ist, und einen nahen Griffabschnitt (31) auf einer abschließenden freien Seite nahe am Griff hat;
Der distale Griffabschnitt (21) ist dünner als der nahe Griffabschnitt (31), der distale Griffabschnitt (21) wird durch eine Verbindung zwischen dem distalen Griffabschnitt (21) und dem Messer (60) allmählich dicker, der Griff hat eine bauchige Seite, die den internen Bereich des Griffs darstellt, und sich vor dem Messer befindet, wenn das Messer zusammengeklappt ist; und eine Rückseite gegenüber der bauchigen Seite;
das freie nahe Ende des nahen Griffabschnitts (31) hat einen wulstigen Abschnitt (33) auf seiner bauchigen Seite,
und ist **dadurch gekennzeichnet, dass** er außerdem Folgendes enthält:
einen Winkelabschnitt mit Stufen (40) zwischen dem distalen Griffabschnitt (21) und dem nahen Griffabschnitt (31);
Der genannte Winkelabschnitt mit Stufen (40) ist nur auf der Rückseite des Griffs geformt; der Winkelabschnitt mit Stufen (40) dient dazu, mit den Fingern oder der Handfläche eines Bedieners angefasst zu werden, um Kraft auf den Griff (20) auszuüben.

2. Kehlkopfdilatator (11) gemäß Patentanspruch 1, in dem
eine erste Leuchtquelle (36) mit dem wulstigen Abschnitt des nahen Griffabschnitts (31) ausgerichtet ist.

3. Kehlkopfdilatator (11) gemäß Patentanspruch 2, in dem
eine zweite Leuchtquelle (26) nahe mit einem Teil auf der bauchigen Seite des distalen Griffabschnitts (21) ausgerichtet ist, der mit dem Messer (60) verbunden ist.

4. Kehlkopfdilatator (11) gemäß Patentanspruch 3, in dem
das Messer (60) aus einem Lichtführungskörper besteht,
Der Griff (20) hat eine dritte Leuchtquelle (66) vor dem Messer (60), um Licht im Messer (60) auszustrahlen, sodass das im Messer (60) ausgestrahlte Licht von einer peripheren Oberfläche des Messers (60) entladen werden kann.

5. Kehlkopfdilatator (11) gemäß Patentanspruch 4, in dem
das Messer (60) mit dem Griff (20) vereint ist, sodass das Messer (60) abnehmbar oder zusammenklappbar ist,
mindestens eine der Leuchtquellen von der ersten bis zur dritten (36, 26, 66) ist eingeschaltet, wenn das Messer (60) im Gebrauchszustand aufrecht auf dem Griff (20) angebracht wird, und
die Leuchtquellen vor der dritten (36, 26, 66) sind ausgeschaltet, wenn das Messer (60) vom Griff (20) abgenommen oder zusammengeklappt wird.

6. Kehlkopfdilatator (11) gemäß einem beliebigen der Patentansprüche von 3 bis 5, in dem
der nahe Griffabschnitt (31) ein Speisegerät (37) enthält.

## Revendications

1. Dilatateur de larynx (11) comprenant:
un manche (20) en forme d'arc ; et
une lame (60), où
le manche(20) a une section de prise distale (21) reliée à la lame (60) et une section de prise rapprochée (31) sur un côté terminal libre près du manche ;
la section de prise distale (21), qui est plus mince que la section de prise rapprochée (31), s'épaissit graduellement sur le raccord entre celle-ci (21) et la lame (60) ; le manche a un côté ventru qui constitue sa périphérie interne et se retrouve devant la lame quand celle-ci est repliée ; il possède aussi un côté arrière opposé au côté ventru ;
l'extrémité libre proche de la section de prise rapprochée (31) a une section gonflée (33) sur son côté ventru,
**caractérisé en ce qu'**il comprend :
une section angulaire à gradins (40) entre la section de prise distale (21) et la section de prise rapprochée (31) ;
la section angulaire à gradins (40) est formée seulement sur le côté arrière du manche ; la section angulaire à gradins (40) peut être saisie par les doigts ou la paume de la main de l'opérateur pour appliquer une force sur le manche (20).

2. Dilatateur de larynx (11), selon la revendication 1, où
une première source lumineuse (36) est alignée dans la section gonflée de la section de prise rapprochée (31).

3. Dilatateur de larynx (11), selon la revendication 2, où
une seconde source lumineuse (26) est alignée prés d'une portion sur le côté ventru de la section de prise distale (21) reliée à la lame (60).

4. Dilatateur de larynx (11), selon la revendication 3, où
la lame (60) est composée d'un corps de guidage de la lumière,
le manche (20) possède une troisième source de lumière (66) qui se trouve devant la lame (60) pour éclairer cette dernière de sorte que la lumière émise sur la lame (60) se répande sur la surface périphérique de la lame (60).

5. Dilatateur de larynx (11), selon la revendication 4, où
la lame (60) est unie au manche (20) de sorte qu'elle puisse être détachée ou repliée,
au moins l'une des sources lumineuses de la première à la troisième (36, 26, 66) est allumée quand la lame (60) se trouve en position droite sur le manche (20) et qu'elle est utilisée, et
les sources lumineuses de la première à la troisième (36, 26, 66) sont éteintes quand la lame (60) est détachée du manche (20) ou repliée.

6. Dilatateur de larynx (11), selon l'une des revendications de 3 à 5, où
la section de prise rapprochée (31) incorpore un alimentateur (37).
